# FASCICULE DE BREVET EUROPEEN

(11) **EP 3 592 332 B1**
(45) Date de publication et mention de la délivrance du brevet: **07.06.2023**
(21) Numéro de dépôt: 18713312.9
(22) Date de dépôt: 06.03.2018
(51) Int. Cl.: A61K 9/00, A61K 9/08, A61K 31/195, C08L 5/08

(54) **NOUVELLE SOLUTION VISCO-ÉLASTIQUE ET SON UTILISATION EN RHUMATOLOGIE**
NEUARTIGE VISKOELASTISCHE LÖSUNG UND VERWENDUNG DAVON IN DER RHUMATOLOGIE
NOVEL VISCOELASTIC SOLUTION AND USE THEREOF IN RHUMATOLOGY

(30) Priorité: 07.03.2017 FR 1751823
(43) Date de publication de la demande: 15.01.2020
(73) Titulaire: Laboratoire de Rhumatologie Appliquée, 69001 Lyon (FR)
(72) Inventeur: SAC-EPEE, Patrick, 69380 Marcilly d'Azergues (FR); SCHOUFT, Alain, 69007 Lyon (FR); CONROZIER, Thierry, 90000 Belfort (FR)
(74) Mandataire: Germain Maureau
(86) Numéro de dépôt international: PCT/FR2018/050499
(87) Numéro de publication internationale: WO 2018/162830

(56) Documents cités:
- EP-A2- 2 173 324
- WO-A2-2012/141536
- RU-C1- 2 128 030
- US-A1- 2014 088 039

## Description

La présente invention a pour objet une solution viscoélastique comprenant un polysaccharide et un agent antifibrinolytique, et l'utilisation de cette solution en rhumatologie.

Une articulation, en anatomie, correspond à la structure qui permet de relier deux os et de leur donner une mobilité l'un par rapport à l'autre. Outre les deux os qui la composent une articulation est constituée par du cartilage hyalin qui recouvre les surfaces osseuses, une membrane synoviale, tapissant une capsule fibreuse, le tout étant stabilisé par un système musculo-ligamentaire. Une articulation diarthrodiale se caractérise par la présence de synovie, un liquide biologique produit par la membrane synoviale, qui facilite le mouvement, absorbe les chocs et protège le cartilage articulaire de l'érosion.

Le liquide synovial est un dialysat de plasma, composé d'électrolytes, de glucose, de protéines, de glycoprotéines et d'acide hyaluronique. L'acide hyaluronique est synthétisé *in situ* par deux types de cellules : les synoviocytes et les chondrocytes. C'est l'acide hyaluronique qui confère au liquide synovial ses propriétés viscoélastiques, indispensables au bon fonctionnement de l'articulation.

L'arthrose est une maladie dégénérative des articulations liée à une dégradation progressive de la matrice cartilagineuse qui se produit sous la dépendance de multiples facteurs dont les principaux sont l'âge, l'excès de contraintes mécaniques (excès de poids, traumatisme, défaut d'axe), certains facteurs métaboliques (syndrome métabolique, diabète de type II, obésité) et la prédisposition génétique.

En cas d'arthrose ou en réponse à des surcharges mécaniques anormales, le cartilage subit des transformations précoces que les chondrocytes tentent de réparer par la synthèse de facteur de croissance, notamment le TGF β et l'IGF1. Les chondrocytes activés secrètent parallèlement d'importantes quantités de protéases, les métalloprotéases matricielles (également désignées par MMPs), dans le cartilage provoquant la dégradation cartilagineuse. Il s'en suit une dérégulation du système plasminogène/plasmine qui stimule encore plus la production de MMPs, créant ainsi le « cercle vicieux » de la dégradation.

Le principal symptôme de l'arthrose est la douleur. Pour soulager cette douleur, l'injection intra-articulaire de corticoïdes est notamment utilisée depuis plus de soixante ans. Néanmoins, si ces injections permettent de soulager les douleurs inflammatoires articulaires, leur efficacité est de courte durée et elles ne peuvent être répétées au-delà de trois ou quatre fois par an, notamment du fait des effets indésirables associés.

De ce fait, des recherches ont été conduites en vue d'identifier des traitements alternatifs permettant de soulager efficacement la douleur tout en limitant les désagréments associés à l'utilisation des corticoïdes. Dans le cadre de ces recherches, les traitements dits de « viscosupplémentation » à base d'acide hyaluronique ont été identifiés. La viscosupplémentation consiste à injecter une solution viscoélastique, contenant généralement de l'acide hyaluronique, dans l'articulation arthrosique afin de lubrifier l'articulation et limiter les phénomènes de frottement et donc la douleur associée à la dégradation cartilagineuse. En effet, au cours de l'arthrose du genou, il a été constaté une diminution très significative de l'acide hyaluronique du liquide synovial, tant qualitative que quantitative, par rapport au liquide synovial sain. L'acide hyaluronique forme avec l'eau un gel déformable viscoélastique qui contribue à la lubrification de l'articulation et au bon fonctionnement du cartilage et des structures ligamentocapsulaires.

Deux générations de « viscosuppléments » ont ainsi vu le jour depuis la fin des années 60 :
- les viscosuppléments dits de « première génération » sont constitués uniquement d'acide hyaluronique et ne se différencient les uns des autres que sur des caractéristiques comme le poids moléculaire, la concentration, la structure (linéaire ou réticulée) ou le volume. Ces produits linéaires de poids moléculaire de 0,7 × 10⁶ Da à 2 × 10⁶ Da après stérilisation, de concentration variable de 0,8 à 2,5%, ont des propriétés viscoélastiques directement proportionnelles à leur concentration et à leur poids moléculaire. Les produits réticulés se différencient par le type de réticulation, la concentration et le volume à injecter ; et
- les viscosuppléments dits de « seconde génération » associent une solution d'acide hyaluronique à un polyol (mannitol ou sorbitol) qui, en protégeant la molécule d'acide hyaluronique de la dégradation, a pour objectif d'améliorer les performances de viscosupplémentation.

Bien que l'acide hyaluronique soit désormais largement employé en rhumatologie, l'efficacité des produits demande encore à être améliorée. En effet, des problèmes de stabilité et de durabilité des solutions d'acide hyaluronique injectées dans l'articulation limitent leur effet lubrifiant dans le temps. Il est donc nécessaire, pour soulager efficacement la douleur du patient atteint d'arthrose, d'effectuer des injections intraarticulaires très fréquentes. Il est donc crucial pour améliorer le confort des patients atteints d'arthrose de rallonger la stabilité, et donc le temps de présence dans l'articulation, des viscosuppléments utilisés dans les traitements de viscosupplémentation.

La demande de brevet EP-A-2173324 décrit l'utilisation combinée d'acide hyaluronique et de polyols en vue d'augmenter la résistance aux dégradations du gel d'acide hyaluronique. Ce document ne fait aucune mention de la possible adjonction d'un agent antifibrinolytique en vue d'améliorer la stabilité de l'acide hyaluronique.

Or, il a maintenant été trouvé de façon tout à fait surprenante que l'adjonction d'un agent antifibrinolytique, l'acide tranexamique, permettait d'améliorer significativement la stabilité des viscosuppléments dans l'articulation et de prolonger de manière significative leur effet lubrifiant dans le temps.

Ainsi, la présente invention a pour objet une solution viscoélastique comprenant :
- un polysaccharide choisi parmi l'acide hyaluronique, la chondroitine sulfate, le kératane, le kératane sulfate, le dermatane sulfate, l'héparine, l'héparane sulfate, le chitosan, le xanthane, les alginates et les caraghénanes, ou un de leurs sels ; et
- un agent antifibrinolytique choisi comme étant l'acide tranexamique.

Les solutions viscoélastiques selon la présente invention peuvent être utilisées comme viscosuppléments et présentent une stabilité dans l'articulation significativement améliorée en comparaison des viscosuppléments classiquement utilisés. Leur effet lubrifiant est donc sensiblement prolongé dans le temps.

Dans le cadre de la présente invention :
- on entend par « solution viscoélastique » toute solution présentant des propriétés rhéologiques (viscosité et élasticité) comparables ou supérieures à celle d'un liquide synovial sain. La viscosité d'une solution peut notamment être mesurée à 6 vitesses de cisaillement différentes sur le viscosimètre à plaque conique pour évaluer le comportement non-newtonien du fluide. L'élasticité (linéaire) d'une solution peut quant à elle être mesurée par un essai de résistance au cisaillement oscillatoire de faible amplitude au cours duquel la réponse de la déformation à une petite contrainte de cisaillement sinusoïdale est mesurée pour 10 fréquences comprises entre 100 et 0,1 Hz ;
- on entend par « viscosupplément » toute solution viscoélastique injectable dans une articulation arthrosique dans le but de lubrifier ladite articulation et limiter les phénomènes de frottement et donc la douleur associée à la dégradation cartilagineuse ;
- on entend par « acide tranexamique » l'acide tranexamique sous ses trois formes, à savoir le dérivé synthétique de la lysine avec une fonction acide carboxylique et une fonction amine branchées en 1,4 (para) sur un cyclohexane, le dérivé BOC-acide tranexamique (protection de la fonction amine par le tert-butoxycarbonyle) et le dérivé FMOC-acide tranexamique (protection de la fonction amine par le fluorénylméthoxycarbonyle) ;
- on entend par « xanthane » tout polysaccharide obtenu par fermentation bactérienne de formule C₃₅H₄₉O₂₉ dont la structure est un assemblage de pentasaccharides constitué par la combinaison d'unités de glucose, de mannose et des dérivés de ces molécules ;
- on entend par « alginates » tout sel hydrosoluble de l'acide alginique avec des métaux alcalins tels que le sodium (également désigné par alginate de sodium), le potassium, le lithium, les cations d'amine inférieures et d'ammonium substitués tels que la méthylamine, l'ethanolamine, la diethanolamine, la triethanolamine ;
- on entend par « caraghénanes » tout polysaccharide linéaire sulfaté extrait d'algues rouges dont la structure chimique est représentée par un enchaînement de molécules de galactoses et d'anhydro-galactose formant des motifs disaccharidiques ou D-galactopyranoses ; et
- on entend par « sel » tout sel d'addition avec un acide minéral ou organique par action d'un tel acide au sein d'un solvant organique ou aqueux tel qu'un alcool, une cétone, un éther ou un solvant chloré, et qui soit acceptable d'un point de vue pharmaceutique. A titre d'exemple de tels sels, on peut citer les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, palmoate, phosphate, salicylate, sulfate, tartrate, théophyllinacétate et p-toluènesulfonate.

La solution viscoélastique selon la présente invention contient donc un polysaccharide tel que défini précédemment et un agent antifibrinolytique choisi comme étant l'acide tranexamique. De préférence, la présente invention a pour objet une solution viscoélastique présentant les caractéristiques suivantes, prises seules ou en combinaison :
- le polysaccharide est choisi comme étant l'acide hyaluronique ou l'un de ses sels. De préférence, le polysaccharide est choisi comme étant l'acide hyaluronique, le hyaluronate de sodium ou le hyaluronate de zinc. De préférence encore, le polysaccharide est choisi comme étant le hyaluronate de sodium ;
- la masse moléculaire du polysaccharide varie de 10 à 4500 kDa ; de préférence de 500 à 4000 kDa ; de préférence encore de 3000 à 3500 kDa ;
- la solution contient de 0,1 à 100 mg/ml de polysaccharide ; de préférence de 1 à 70 mg/ml de polysaccharide ; de préférence encore 10 à 30 mg/ml de polysaccharide ; et/ou
- la solution contient de 0,1 à 100 mg/ml d'acide tranexamique ; de préférence de 1 à 70 mg/ml d'acide tranexamique ; de préférence encore de 10 à 50 mg/ml d'acide tranexamique.

Comme exemples de solutions viscoélastiques selon l'invention, on peut notamment citer :
- une solution viscoélastique comprenant un polysaccharide choisi comme étant l'acide hyaluronique et l'acide tranexamique ;
- une solution viscoélastique comprenant un polysaccharide choisi comme étant la chondroitine sulfate et l'acide tranexamique ;
- une solution viscoélastique comprenant un polysaccharide choisi comme étant le kératane et l'acide tranexamique ;
- une solution viscoélastique comprenant un polysaccharide choisi comme étant le kératane sulfate et l'acide tranexamique ;
- une solution viscoélastique comprenant un polysaccharide choisi comme étant le dermatane sulfate et l'acide tranexamique ;
- une solution viscoélastique comprenant un polysaccharide choisi comme étant l'héparine et l'acide tranexamique ;
- une solution viscoélastique comprenant un polysaccharide choisi comme étant le l'héparane sulfate et l'acide tranexamique ;
- une solution viscoélastique comprenant un polysaccharide choisi comme étant le chitosan et l'acide tranexamique ;
- une solution viscoélastique comprenant un polysaccharide choisi comme étant le xanthane et l'acide tranexamique ;
- une solution viscoélastique comprenant un polysaccharide choisi comme étant les alginates et l'acide tranexamique ; ou
- une solution viscoélastique comprenant un polysaccharide choisi comme étant les caraghénanes et l'acide tranexamique.

La solution viscoélastique peut en outre contenir un polyol, lequel permet d'améliorer encore la protection de l'acide hyaluronique contre la dégradation dans l'articulation. Ainsi, la présente invention a également pour objet une solution viscoélastique telle que définie précédemment, ladite solution contenant en outre un polyol.

De préférence, ledit polyol est préférence choisi comme étant le glycérol, le propylène glycol, le sorbitol, le mannitol ou le xylitol. De préférence encore le polyol est choisi comme étant le mannitol ou le sorbitol.

De préférence, la solution selon la présente invention contient de 0,1 à 100 mg/ml de polyol, de préférence de 1 à 70 mg/ml de polyol, de préférence encore de 5 à 50 mg/ml de polyol.

La solution viscoélastique selon la présente invention est administrée par voie intra-articulaire. Pour ce faire, la solution peut se présenter sous toute forme adaptée à une telle administration. De préférence, la solution selon la présente invention est contenue dans un flacon en verre ou en polymère plastique de 0,1 à 20 ml. De préférence encore, la solution est contenue dans une seringue prête à l'emploi.

La solution viscoélastique selon la présente invention est administrée dans des volumes variables en fonction de l'articulation concernée et de l'avancement de sa détérioration. De préférence, la solution selon la présente invention est administrée dans des volumes variant de 0,1 ml à 10 ml

La solution viscoélastique selon la présente invention peut être administrée selon un calendrier continu ou non et à tout moment de la journée. De préférence, la solution selon la présente invention est administrée tous les sept à vingt-huit jours, de préférence encore tous les sept jours. La durée de traitement sera affinée en fonction du patient et de l'intensité des symptômes.

La solution viscoélastique selon la présente invention peut donc être utilisée comme viscosupplément. Ainsi, la présente invention a également pour objet une solution viscoélastique telle que définie précédemment pour utilisation comme viscosupplément.

Compte tenu des propriétés de la solution viscoélastique selon la présente invention, d'autres utilisations de celle-ci peuvent également être envisagées. Ainsi, la solution viscoélastique selon la présente invention peut également être utilisée
- en médecine esthétique pour le comblement des rides et des sillons ;
- en ophtalmologie pour la protection, la lubrification ou le soutien de cellules ou de tissus lors d'interventions chirurgicales sur l'oeil comme par exemple la chirurgie de la cataracte ou du glaucome, les greffes de cornée ou, la pose d'implants intraoculaires ; ou
- en urologie/gynécologie comme gel permettant, par exemple, d'augmenter le volume du sphincter ou de l'urètre, de lubrifier les parois vaginales ou de réduire les adhérences cellulaires/tissulaires.

La présente invention est illustrée de manière non limitative par les exemples suivants.

### Exemple 1 - Préparation d'une solution viscoélastique selon l'invention

Le procédé suivant permet de préparer les solutions viscoélastiques A à E dont la composition est rapportée dans les tableaux 1 à 5 ci-dessous.

On prépare une solution de 125 ml de tampon phosphate dont le pH varie entre 7.0 et 7.3 dont on ajuste l'osmolarité à 260 à 320 mOsm/kg par ajout de sel de NaCl. On ajoute à cette solution tampon la quantité désirée d'acide tranexamique puis d'acide hyaluronique et on mélange jusqu'à obtention d'une solution visqueuse homogène.

**Tableau 1 - Solution viscoélastique A**

| **Ingrédient** | **Quantité (en mg/ml)** |
|---|---|
| Hyaluronate de sodium | 18 |
| Acide tranexamique | 10 |

**Tableau 2 - Solution viscoélastique 8**

| **Ingrédient** | **Quantité (en mg/ml)** |
|---|---|
| Hyaluronate de sodium | 18 |
| Acide tranexamique | 20 |

**Tableau 3 - Solution viscoélastique C**

| **Ingrédient** | **Quantité (en mg/ml)** |
|---|---|
| Hyaluronate de sodium | 18 |
| Acide tranexamique | 30 |

**Tableau 4 - Solution viscoélastique D**

| **Ingrédient** | **Quantité (en mg/ml)** |
|---|---|
| Hyaluronate de sodium | 18 |
| Acide tranexamique | 40 |

**Tableau 5 - Solution viscoélastique E**

| **Ingrédient** | **Quantité (en mg/ml)** |
|---|---|
| Hyaluronate de sodium | 22 |
| Acide tranexamique | 15 |

### Exemple 2 - Résistance à la dégradation des solutions de l'invention

On compare la résistance à la dégradation par la hyaluronidase dans le temps d'une solution viscoélastique selon l'invention (solution E - tableau 5) avec une solution viscoélastique de référence sans acide tranexamique (Référence 1 - Tableau 6).

**Tableau 6-Solui-ion viscoélastique classique (Référence 1)**

| **Ingrédient** | **Quantité (en mg/ml)** |
|---|---|
| Hyaluronate de sodium | 22 |
| *Acide tranexamique* | 0 |

Pour ce faire, on mesure l'évolution de la viscosité des deux solutions viscoélastiques dans le temps selon le protocole suivant :
- on dissout 5.8 mg d'enzyme (hyaluronidase Sigma from sheep testes, Type III, lyophilized powder, ≥500 U/mg) dans 5ml de tampon phosphate PBS pH=7.3 (DPBS Tampon Gibco by Life Technologies).
- on mesure la cinétique de dégradation de l'acide hyaluronique dans la solution E et dans la solution référence 1 par mesure de la viscosité à 1s⁻¹.

Les essais ont été réalisés avec 5 µl de solution enzymatique pour 3ml de solution E ou de Référence 1.

La pente de la courbe de dégradation enzymatique après ajout de 5 µl de hyaluronidase a ainsi été mesurée. Les résultats sont rapportés dans le Tableau 7 suivant.

**Tableau 7 - Pente de la courbe de dégradation enzymatique après ajout de hyaluronidase**

| | **Test 1** | **Test 2** |
|---|---|---|
| **Référence 1** | 0,019 | 0,015 |
| **Solution E** | 0,0041 | 0,0042 |

Comme le montrent les résultats précédents, la pente de dégradation de l'acide hyaluronique est trois fois plus faible en présence d'acide tranexamique 15 mg/ml (moyenne : 0,0042 versus 0,015). Cela indique que l'acide tranexamique protège l'acide hyaluronique de façon efficace contre les dégradations par la hyaluronidase et augmente le temps de présence et la stabilité de l'acide hyaluronique. La solution selon la présente invention (formulation E). La solution selon la présente invention est donc beaucoup plus stable et efficace dans le temps que les solutions classiquement utilisées.

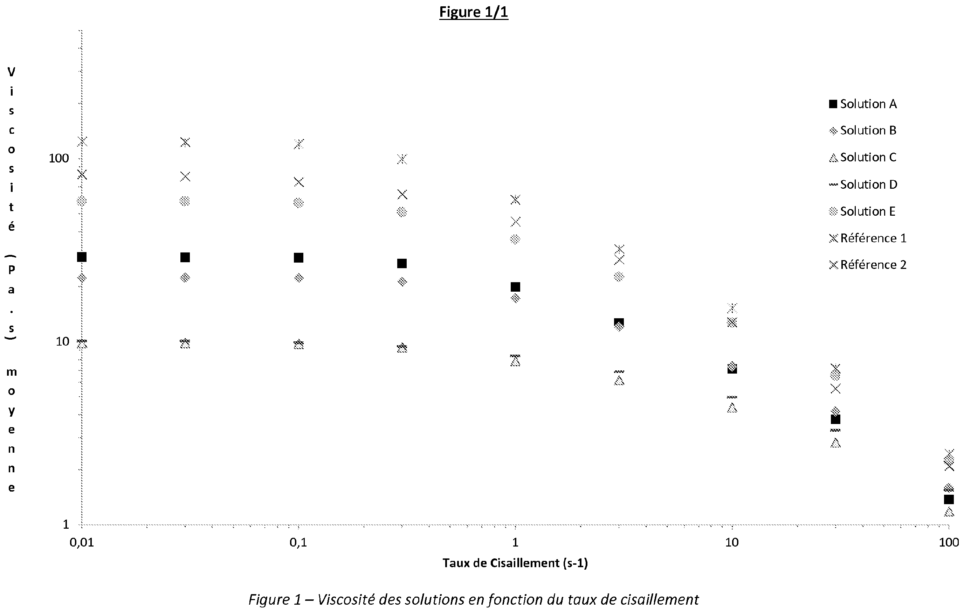

## Revendications

1. Solution viscoélastique comprenant :
- un polysaccharide choisi parmi l'acide hyaluronique, la chondroitine sulfate, le kératane, le kératane sulfate, le dermatane sulfate, l'héparine, l'héparane sulfate, le chitosan, le xanthane, les alginates et les caraghénanes, ou un de leurs sels ; et
- un agent antifibrinolytique choisi comme étant l'acide tranexamique.

2. Solution selon la revendication 1, **caractérisée en ce que** le polysaccharide est choisi comme étant l'acide hyaluronique ou l'un de ses sels.

3. Solution selon la revendication 1 ou 2, **caractérisée en ce qu'**elle contient de 0,1 à 100 mg/ml de polysaccharide.

4. Solution selon la revendication 3, **caractérisée en ce qu'**elle contient de 10 à 30 mg/ml de polysaccharide.

5. Solution selon l'une quelconque des revendications 1 à 4, **caractérisée en ce qu'**elle contient de 0,1 à 100 mg/ml d'agent antifibrinolytique.

6. Solution selon la revendication 5, **caractérisée en ce qu'**elle contient de 10 à 50 mg/ml d'agent antifibrinolytique.

7. Solution selon l'une quelconque des revendications 1 à 6, **caractérisée en ce qu'**elle contient en outre un polyol choisi comme étant le glycérol, le propylène glycol, le sorbitol, le mannitol ou le xylitol, de préférence encore le mannitol ou le sorbitol.

8. Solution selon la revendication 7, **caractérisée en ce qu'**elle contient de 0,1 à 100 mg/ml de polyol.

9. Solution selon l'une des revendications 1 à 8 pour utilisation comme viscosupplément.

## Patentansprüche

1. Viskoelastische Lösung, umfassend:
- ein Polysaccharid, das aus Hyaluronsäure, Chondroitinsulfat, Keratan, Keratansulfat, Dermatansulfat, Heparin, Heparansulfat, Chitosan, Xanthan, Alginaten und Carrageenanen oder einem ihrer Salze ausgewählt ist; und
- einem Antifibrinolytikum, das als Tranexamsäure ausgewählt ist.

2. Lösung nach Anspruch 1, **dadurch gekennzeichnet, dass** das Polysaccharid als Hyaluronsäure oder eines ihrer Salze ausgewählt ist.

3. Lösung nach Anspruch 1 oder 2, **dadurch gekennzeichnet, dass** sie 0,1 bis 100 mg/ml Polysaccharid enthält.

4. Lösung nach Anspruch 3, **dadurch gekennzeichnet, dass** sie 10 bis 30 mg/ml Polysaccharid enthält.

5. Lösung nach einem der Ansprüche 1 bis 4, **dadurch gekennzeichnet, dass** sie 0,1 bis 100 mg/ml Antifibrinolytikum enthält.

6. Lösung nach Anspruch 5, **dadurch gekennzeichnet, dass** sie 10 bis 50 mg/ml Antifibrinolytikum enthält.

7. Lösung nach einem der Ansprüche 1 bis 6, **dadurch gekennzeichnet, dass** sie weiterhin ein Polyol enthält, das als Glycerin, Propylenglykol, Sorbit, Mannit oder Xylit, mehr bevorzugt Mannit oder Sorbit ausgewählt ist.

8. Lösung nach Anspruch 7, **dadurch gekennzeichnet, dass** sie 0,1 bis 100 mg/ml Polyol enthält.

9. Lösung nach einem der Ansprüche 1 bis 8 zur Verwendung als Viskosupplement.

## Claims

1. A viscoelastic solution comprising:
- a polysaccharide chosen among hyaluronic acid, chondroitin sulfate, keratan, keratan sulfate, dermatan sulfate, heparin, heparan sulfate, chitosan, xanthan, alginates and carrageenans, or one of the salts thereof; and
- an antifibrinolytic agent chosen as being tranexamic acid.

2. The solution according to claim 1, **characterised in that** the polysaccharide is chosen as being hyaluronic acid or one of the salts thereof.

3. The solution according to claim 1 or 2, **characterised in that** it contains from 0.1 to 100 mg/ml of polysaccharide.

4. The solution according to claim 3, **characterised in that** it contains from 10 to 30 mg/ml of polysaccharide.

5. The solution according to any one of claims 1 to 4, **characterised in that** it contains from 0.1 to 100 mg/ml of an antifibrinolytic agent.

6. The solution according to claim 5, **characterised in that** it contains from 10 to 50 mg/ml of an antifibrinolytic agent.

7. The solution according to any one of claims 1 to 6, **characterised in that** it further contains a polyol chosen as being glycerol, propylene glycol, sorbitol, mannitol or xylitol, more preferably mannitol or sorbitol.

8. The solution according to claim 7, **characterised in that** it contains from 0.1 to 100 mg/ml of polyol.

9. The solution according to any of claims 1 to 8 for use as a viscosupplement
